# EUROPÄISCHE PATENTSCHRIFT

(11) **EP 1 420 007 B1**
(45) Veröffentlichungstag und Bekanntmachung des Hinweises auf die Patenterteilung: **02.11.2006**
(21) Anmeldenummer: 03103465.5
(22) Anmeldetag: 19.09.2003
(51) Int. Cl.: C07C 213/08, C07C 215/08

(54) **Verfahren zur Herstellung von Lösungen von Alkalimetallsalzen funktionalisierter Alkohole**
Process for the preparation of alkali metal salts of functionalised alcohols
Procédé de fabrication de solutions de sels de métaux alcalins d' alcools fonctionnalisés

(30) Priorität: 12.11.2002 DE 10252413
(43) Veröffentlichungstag der Anmeldung: 19.05.2004
(73) Patentinhaber: Degussa AG, 40474 Düsseldorf (DE)
(72) Erfinder: Ruwwe, Johannes, 53859, Niederkassel (DE); Stadtmüller, Klaus, 63755, Alzenau (DE)

(56) Entgegenhaltungen:
- EP-A- 0 518 013

## Beschreibung

Die vorliegende Erfindung betrifft ein Verfahren zur Herstellung von Lösungen von Alkalimetallsalzen funktionalisierter Alkohole.

Alkalimetallsalze funktionalisierter Alkohole finden Anwendung in verschiedenen Bereichen, zum Beispiel bei der Herstellung von kupferhaltigen Filmen, bei denen, wie in US 5 034 248 beschrieben, das Natriumsalz des 2-Amino-1-ethanols oder das Natriumsalz des 2-Methylamino-1-ethanols eingesetzt wird. Ebenfalls können solche Alkalimetallsalze funktionalisierter Alkohole zur Herstellung pharmazeutischer Produkte dienen, wie zum Beispiel die Natriumsalze des 2-Ethylamino-1-ethanols und des 2-Amino-1-butanols, welche in US 4 235 902 beschrieben sind, oder das Natriumsalz des 2-Amino-1-ethanols, welches in US 4 372 974 beschrieben ist. Eine weitere Anwendung des Natriumsalzes des 2-Amino-1-ethanols ist in JP 063 061 024 beschrieben, wo es zur Herstellung von Polyphosphazenen eingesetzt wird.

Die Herstellung von Alkalimetallsalzen funktionalisierter Alkohole kann beispielsweise durch Umsetzung der freien Alkohole mit Alkalimetallen erfolgen, wie zum Beispiel in US 4 235 902 oder in US 4 372 974 beschrieben. Die Umsetzung von funktionalisierten Alkoholen mit Alkalimetallen kann ebenfalls in flüssigem Ammoniak als Lösemittel erfolgen. Der Nachteil dieser Verfahren besteht in der Verwendung des sehr reaktiven metallischen Natriums, für dessen Einsatz aufwändige Sicherheitsvorkehrungen getroffen werden müssen.

Eine weitere Möglichkeit zur Darstellung solcher Alkalimetallsalze besteht in der Umsetzung von freien Alkoholen mit Alkalimetallhydriden. Eine solche Umsetzung zur Herstellung des Natriumsalzes des 2-Amino-1-ethanols ist in GB 1 341 375 beschrieben, wobei dieses zur Herstellung von Heterocyclen verwendet wird. Nachteilig an diesem Verfahren ist, dass das teure Natriumhydrid eingesetzt werden muss.

Es besteht weiterhin die Möglichkeit, funktionalisierte Alkohole mit Alkalimetallamiden oder metallorganischen Verbindungen der Alkalimetalle umzusetzen, um die Alkalimetallsalze der funktionalisierten Alkohole zu erhalten. Solche Umsetzungen sind in US 5 629 452 beschrieben, wobei Diphenylmethylkalium, Diphenylmethylnatrium oder Natriumamid eingesetzt wird. Diese Verbindungen sind aufwändig herzustellen oder aber teuer. Desweiteren verbleibt, zumindest im Fall des Diphenylmethylkaliums und des Diphenylmethylnatriums, nach der Umsetzung das entstehende Diphenylmethan in der erhaltenen Reaktionsmischung, wenn kein weiterer Aufarbeitungsschritt durchgeführt wird.

In der EP 0 518 013 wird ein Verfahren zur Herstellung von 1-Alkoxy-2-dialkylaminoethanen beschrieben. Im ersten Schritt werden dabei die entsprechenden Aminoalkohole durch Umsetzen mit Alkalisalzen niederer Alkohole in die jeweiligen Alkalimetallsalze der funktionalisierten Alkohole überführt. Die Reaktion wird dadurch vervollständigt, dass der entstehende niedere Alkohol aus dem Reaktionsgemisch entfernt wird, wobei vor der destillativen Aufarbeitung ein Schleppmittel zur Entfernung des niederen Alkohols zur Mischung hinzugegeben wird. Alternativ schlägt die EP 0 518 013 jedoch auch vor, als Schleppmittel die jeweiligen Endprodukte der Ethersynthese heranzuziehen. Sofern man jedoch daran denkt, die Alkalimetallsalze der funktionalisierten Alkohole anders als zur Etherbildung einzusetzen, verbietet sich dieses Vorgehen.

Aufgabe der vorliegenden Erfindung war daher die Angabe eines weiteren Verfahrens zur Herstellung von Alkalimetallsalzen funktionalisierter Alkohole, welches insbesondere im technischen Maßstab unter ökonomischen und ökologischen Gesichtspunkten vorteilhaft anzuwenden und darüber hinaus in Bezug auf die Raum/Zeit-Ausbeute den Verfahren des Standes der Technik überlegen ist.

Die Aufgabe wird anspruchsgemäß gelöst.

Dadurch, dass man in einem Verfahren zur Herstellung von Lösungen von Alkalimetallsalzen der allgemeinen Formel (I)
MOYXR¹ (I)
worin
M steht für Li, Na, K, Rb oder Cs,
Y steht für (C₁-C₈)-Alkylen,
X steht für O, S, NR¹,
R¹ steht für H, (C₁-C₈)-Alkyl, (C₂-C₈)-Alkenyl, (C₂-C₈)-Alkinyl, (C₃-C₈)-Cycloalkyl, (C₆-C₁₈)-Aryl, (C₇-C₁₉)-Aralkyl, (C₃-C₁₈)-Heteroaryl, (C₄-C₁₉)-Heteroaralkyl, ((C₁-C₈)-Alkyl)₁₋₃-(C₃-C₈)-Cycloalkyl, ((C₁-C₈)-Alkyl)₁₋₃-(C₆-C₁₈)-Aryl, ((C₁-C₈)-Alkyl)₁₋₃-(C₃-C₁₈)-Heteroaryl, in einem Lösemittelgemisch aufweisend den korrespondierenden Alkohol der allgemeinen Formel (I) die Umsetzung des korrespondierenden Alkohols der allgemeinen Formel (I) mit dem entsprechenden Alkalimetallsalz eines Alkohols der allgemeinen Formel (II)
MOR² (II)
worin
M die oben angegebene Bedeutung annimmt und
R² bedeutet Methyl, Ethyl, Propyl, Isopropyl, sec-, Iso- oder tert.-Butyl, durchführt, oder die Umsetzung des korrespondierenden Alkohols der allgemeinen Formel (I) mit dem entsprechenden Alkalimetallhydroxid betreibt, wobei zuerst eine Teilmenge des freigesetzten Alkohols der allgemeinen Formel (II) oder des freigesetzten Wassers ohne Zusatz eines Schleppmittels und anschließend die Restmenge unter Zuhilfenahme mindestens eines weiteren organischen Lösemittels als Schleppmittel aus der Lösung destilliert wird, gelangt man völlig überraschend, dafür aber nicht minder vorteilhaft, zur Lösung der gestellten Aufgabe. In unvorhersehbarer Weise lässt sich der niedere Alkohol bzw. das Wasser aus der Reaktionsmischung ohne Schleppmittelzugabe bis zu einem gewissen Teil herausdestillieren, ohne dass Nebenreaktionen in der relativ konzentrierteren Mischung ablaufen, die die Ausbeute und die Produktreinheit beeinträchtigen würden. Durch die geringere Destillatmenge verringert sich darüber hinaus die Destillationszeit, was einerseits die Raum/Zeit-Ausbeute steigern hilft und andererseits die thermische Belastung der Produkte bei der Destillation verringert.

Als bevorzugte Verbindungen der allgemeinen Formel (I) werden Alkalimetallsalze von Alkoholen, in denen M steht für
Li, Na, K, Rb oder Cs,
Y steht für (C₁-C₈)-Alkylen,
X steht für NR¹,
R¹ steht für H, (C₁-C₈)-Alkyl, verwendet.

Ganz besonders bevorzugt sind Verbindungen ausgewählt aus der Gruppe der aliphatischen Amino-Alkohole, z. B. bestehend aus 2-Amino-ethanol.

Als Alkoholat (II) können im oben genannten Rahmen alle dem Fachmann für diesen Zweck in Frage kommenden Alkoholate herangezogen werden. Vorzugsweise wird das kostengünstige Alkalimetallsalz des Methanols oder Ethanols zur Deprotonierung genommen. Die Alkoholate können dabei als ihre Lösungen oder in festem Zustand eingesetzt werden. Vorzugsweise werden die Salze in festem Zustand angewandt, um den Destillationsmittelstrom so gering wie möglich zu halten und eine weitere Vermengung unterschiedlicher Lösemittel zu vermeiden.

Prinzipiell ist der Fachmann frei in der Wahl der Schleppmittel, sofern sie sich zum gewünschten Zweck eignen, d. h. sie sollten möglichst einen Siedepunkt besitzen, der größer als der des freigesetzten Alkohols R²OH bzw. H₂O ist, oder möglichst viel des abzutrennenden Alkohols oder Wassers aus dem Reaktionsgemisch durch Azeotropbildung ausschleusen können. Darüber hinaus ist es vorteilhaft, ein Schleppmittel zu verwenden, welches sich als inert erweist, damit Reste dieses Schleppmittels bedenkenlos im Reaktionsgemisch verbleiben können, ohne dass eine folgende Reaktion der Alkalimetallsalze der funktionalisierten Alkohole negativ beeinträchtigt wird.

Als weiteres organisches Lösemittel kann ein Schleppmittel verwendet werden, das ausgewählt ist aus der Gruppe, bestehend aus linearen oder cyclischen aliphatischen Kohlenwasserstoffen, aromatischen Kohlenwasserstoffen, Verbindungen des Typs der allgemeinen Formel (III)
R¹(XY)ₙXR¹ (III)
worin
n = 1 oder 2 sein kann,
R¹ jeweils unabhängig voneinander, X jeweils unabhängig voneinander und Y die in Anspruch 1 angegebene Bedeutung annehmen,
und Alkoholen der allgemeinen Formel (IV)
R³OH (IV)
worin
R³ = (C₄-C₈)-Alkyl, (C₂-C₈)-Alkenyl, (C₂-C₈)-Alkinyl oder (C₃-C₈)-Cycloalkyl bedeuten.

Bevorzugt ist die Verwendung von Schleppmitteln aus der Gruppe bestehend aus Toluol, Xylol, Benzol, Hexan, Heptan, Cyclohexan, Methylcyclohexan, oder weniger acide Alkohole wie z. B. n-Butanol oder Ethylenglykol oder Ethylendiamin oder Diethylentriamin.

Der entstehende Alkohol (II) bzw. das Reaktionswasser mit bzw. ohne Schleppmittel wird vorteilhafterweise in einem Druckbereich von 10 mbar bis 15 bar destillativ aus der Reaktionsmischung abgezogen. Bevorzugt erfolgt die Destillation jedoch unter Unterdruck, ganz besonders bevorzugt zwischen 100 und 500 mbar oder darunter, je nach technischer Machbarkeit.

Kern des vorliegenden Verfahrens ist die Tatsache, dass das zur Deprotonierung eingesetzte Alkalialkoholat oder das Alkalihydroxid nach der Umsetzung als leichtsiedender Alkohol bzw. Wasser aus dem Reaktionsgemisch zuerst ohne Zusatz eines Schleppmittels abdestilliert werden. Es bleibt dem Fachmann überlassen, bis zu welchem Grad diese erste Destillation getrieben wird, doch orientiert man sich hier vorzugsweise an der Wirtschaftlichkeit des Verfahrens bzw. der erhältlichen Produktreinheit, die möglichst hoch ausfallen sollte. Vorzugsweise werden 10 - 90 Vol.-% des freigesetzten Alkohols (II) oder Wassers ohne Schleppmittel abdestilliert, bevor zur weiteren Destillation die entsprechenden Schleppmittel zum Reaktionsgemisch hinzugegeben werden. Unter den oben genannten Prämissen erscheint eine möglichst weitgehende Entfernung der abzudestillierenden Bestandteile (Wasser, Alkohol) im ersten Destillationsschritt jedoch als besonders bevorzugt.

Im Allgemeinen geht man bei der vorliegenden Erfindung so vor, dass man festes Alkalialkoholat oder Alkalihydroxid zu einem funktionalisierten Alkohol hinzufügt und beginnt, das entstehende Wasser oder den entstehenden Alkohol aus der Mischung unter vermindertem Druck abzudestillieren. Ab einem bestimmten Punkt, der durch Routineexperimente im Hinblick auf oben genannte Sachverhalte zu ermitteln ist, gibt man das Schleppmittel zur Reaktionsmischung hinzu und destilliert solange, bis das Reaktionswasser oder der gebildete Alkohol zu einem erträglichen Maß vermindert ist (häufig unter 100 ppm). Die Ausbeute an Produkt und dessen Reinheit liegen dabei immer > 90 % bis 99 %. Die in Lösung vorliegenden funktionalisierten Alkoholate können so in nachfolgenden Reaktionen wie z. B. einer Etherbildung mit Alkylhalogeniden eingesetzt werden. Aufgrund der Tatsache, dass Reinheit und Ausbeute an Verbindungen der Formel (I) derart gut sind, können auch Nachfolgeprodukte, wie die ins Auge gefassten Ether, in hohen Ausbeuten und entsprechend guten Reinheiten gewonnen werden. Dieses, kombiniert mit einer höheren Raum/Zeit-Ausbeute, ist für den Fachmann im Hinblick auf den Stand der Technik besonders überraschend.

Als (C₁-C₈)-Alkyl sind anzusehen Methyl, Ethyl, n-Propyl, Isopropyl, n-Butyl, Isobutyl, sec-Butyl, tert-Butyl, Pentyl, Hexyl, Heptyl oder Octyl samt aller Bindungsisomeren.

(C₁-C₈)-Alkylen bedeutet einen (C₁-C₈)-Alkylrest, der über zwei Valenzen an das in Frage stehende Molekül gebunden ist. Diese Reste können einfach oder mehrfach mit (C₁-C₈)-Alkoxy, (C₁-C₈)-Haloalkyl, Halogen, NH₂, NH(C₁-C₈)-Alkyl, N[(C₁-C₈)-Alkyl]₂ oder S-(C₁-C₈)-Alkyl substituiert sein.

Als (C₂-C₈)-Alkenyl ist ein wie oben dargestellter (C₁-C₈)-Alkyl-Rest mit Ausnahme von Methyl zu verstehen, der mindestens eine Doppelbindung aufweist.

Unter (C₂-C₈)-Alkinyl ist ein wie oben dargestellter (C₁-C₈)-Alkyl-Rest mit Ausnahme von Methyl zu verstehen, der mindestens eine Dreifachbindung aufweist.

Unter (C₁-C₈)-Acyl versteht man einen über eine -C=O-Funktion ans Molekül gebundenen (C₁-C₈)-Alkyl-Rest.

Unter (C₃-C₈)-Cycloalkyl versteht man Cyclopropyl, Cyclobutyl, Cyclopentyl, Cyclohexyl bzw. Cycloheptylreste etc. Diese können mit einem oder mehreren Halogenen und/oder N-, O-, P-, S-atomhaltigen Resten substituiert sein und/oder N-, O-, P-, S-atomhaltige Reste im Ring aufweisen, wie z. B. 1-, 2-, 3-, 4-Piperidyl, 1-, 2-, 3-Pyrrolidinyl, 2-, 3-Tetrahydrofuryl oder 2-, 3-, 4-Morpholinyl. Dieser kann einfach oder mehrfach mit (C₁-C₈)-Alkoxy, (C₁-C₈)-Haloalkyl, Halogen, NH₂, S-(C₁-C₈)-Alkyl, (C₁-C₈)-Acyl, (C₁-C₈)-Alkyl substituiert sein.

Unter einem (C₆-C₁₈)-Arylrest wird ein aromatischer Rest mit 6 bis 18 C-Atomen verstanden. Insbesondere zählen hierzu Verbindungen wie Phenyl-, Naphthyl-, Anthryl-, Phenanthryl- oder Biphenylreste. Dieser kann einfach oder mehrfach mit (C₁-C₈)-Alkoxy, (C₁-C₈)-Haloalkyl, Halogen, NH₂, S-(C₁-C₈)-Alkyl, (C₁-C₈)-Acyl, (C₁-C₈)-Alkyl substituiert sein.

Ein (C₇-C₁₉)-Aralkylrest ist ein über einen (C₁-C₈)-Alkylrest an das Molekül gebundener (C₆-C₁₈)-Arylrest.

(C₁-C₈)-Alkoxy ist ein über ein Sauerstoffatom an das betrachtete Molekül gebundener (C₁-C₈)-Alkyl-Rest.

(C₁-C₈)-Haloalkyl ist ein mit einem oder mehreren Halogenatomen substituierter (C₁-C₈)-Alkyl-Rest.

Ein (C₃-C₁₈)-Heteroarylrest bezeichnet im Rahmen der Erfindung ein fünf-, sechs- oder siebengliedriges aromatisches Ringsystem aus 3 bis 18 C-Atomen, welches Heteroatome wie z. B. Stickstoff, Sauerstoff oder Schwefel im Ring aufweist. Als solche Heteroaromaten werden insbesondere Reste angesehen, wie 1-, 2-, 3-Furyl, 1-, 2-, 3-Pyrrolyl, 1-,2-,3-Thienyl, 2-, 3-, 4-Pyridyl, 2 -, 3-, 4-, 5-, 6-, 7-Indolyl, 3-, 4-, 5-Pyrazolyl, 2-, 4-, 5-Imidazolyl, Acridinyl, Chinolinyl, Phenanthridinyl, 2-, 4-, 5-, 6-Pyrimidinyl. Dieser kann einfach oder mehrfach mit (C₁-C₈)-Alkoxy, (C₁-C₈)-Haloalkyl, Halogen, NH₂, S-(C₁-C₈)-Alkyl, (C₁-C₈)-Acyl oder (C₁-C₈)-Alkyl substituiert sein.

Unter einem (C₄-C₁₉)-Heteroaralkyl wird ein dem (C₇-C₁₉)-Aralkylrest entsprechendes heteroaromatisches System verstanden.

Als Halogene kommen Fluor, Chlor, Brom und Iod in Frage.

### Beispiele:

Die folgenden Beispiele sollen den Gegenstand der Erfindung näher erläutern.

### Beispiel 1 (erfindungsgemäß):

Benötigt wird das Natriumsalz des 2-Amino-1-ethanols in einem Gemisch aus 2-Amino-1-ethanol und n-Butanol, es ist erforderlich, dass der Methanolgehalt möglichst klein ist:
350 g (5,73 mol) 2-Amino-1-ethanol werden mit 92 g (1,70 mol) festem Natrium-methanolat versetzt. Bei einem Druck von 400 mbar werden 46 g Methanol abdestilliert, was ca. 84 % der theoretischen Menge entspricht. Anschließend werden 199 g n-Butanol zugegeben und bei einem Druck von 150 mbar weitere 115 g Destillat bis zu einer Sumpftemperatur von 110 °C abgenommen. Es kann eine Methanol-Konzentration von nur noch 0,013 % festgestellt werden.

### Beispiel 2 (erfindungsgemäß):

Benötigt wird das Natriumsalz des 2-Amino-1-butanols in einem Gemisch aus 2-Amino-1-butanol und Toluol, es ist erforderlich, dass der Methanolgehalt möglichst klein ist:
350 g (3,93 mol) 2-Amino-1-butanol werden mit 92 g (1,70 mol) festem Natrium-methanolat versetzt. Bei einem Druck von 400 mbar werden 47 g Methanol abdestilliert, was ca. 84 % der theoretischen Menge entspricht. Anschließend werden 200 g Toluol zugegeben und bei einem Druck von 150 mbar weitere 120 g Destillat bis zu einer Sumpftemperatur von 120 °C abgenommen. Es kann eine Methanol-Konzentration von nur noch 0,011 % festgestellt werden.

### Beispiel 3 (erfindungsgemäß):

Benötigt wird das Natriumsalz des 2-Amino-1-butanols in einem Gemisch aus 2-Amino-1-butanol und n-Butanol, es ist erforderlich, dass der Methanolgehalt möglichst klein ist:
350g (3,93 mol) 2-Amino-1-butanol werden mit 92 g (1,70 mol) festem Natrium-methanolat versetzt. Bei einem Druck von 400 mbar werden 47 g Methanol abdestilliert, was ca. 84 % der theoretischen Menge entspricht. Anschließend werden 205 g n-Butanol zugegeben und bei einem Druck von 150 mbar weitere 120 g Destillat bis zu einer Sumpftemperatur von 123 °C abgenommen. Es kann eine Methanol-Konzentration von nur noch 0,011 % festgestellt werden.

### Beispiel 4 (erfindungsgemäß):

Benötigt wird das Natriumsalz des 2-Amino-1-butanols in einem Gemisch aus 2-Amino-1-butanol und n-Butanol, es ist erforderlich, dass der Methanolgehalt möglichst klein ist:
350 g (3,93 mol) 2-Amino-1-butanol werden mit 305 g (1,70 mol) Natrium-methanolat-Lösung (30 %ig in Methanol) versetzt. Bei einem Druck von 150 mbar werden 244 g Methanol abdestilliert, was ca. 90 % der theoretischen Menge entspricht. Anschließend werden 195 g n-Butanol zugegeben und bei einem Druck von 150 mbar weitere 120 g Destillat bis zu einer Sumpftemperatur von 114 °C abgenommen. Es kann eine Methanol-Konzentration von nur noch 0,010 % festgestellt werden.

### Beispiel 5 (Vergleichsbeispiel):

Benötigt wird das Natriumsalz des 2-Amino-1-ethanols in einem Gemisch aus 2-Amino-1-ethanol und Toluol, es ist erforderlich, dass der Methanolgehalt möglichst klein ist:
350 g (5,73 mol) 2-Amino-1-ethanol werden mit 92 g (1,70 mol) festem Natrium-methanolat versetzt. Bei einem Druck von 400 mbar werden an einer Kolonne mit 26 Trennstufen bis zu einer Sumpftemperatur von 171 °C 51 g Methanol abdestilliert, was ca. 94 % der theoretischen Menge entspricht. Bei weiterem Erwärmen des Sumpfes tritt deutliche Verfärbung auf. Anschließend werden 93 g Toluol zugegeben. Der Restmethanolgehalt beträgt 0,71%.

### Beispiel 6 (Vergleichsbeispiel):

Benötigt wird das Natriumsalz des 2-Amino-1-butanols in einem Gemisch aus 2-Amino-1-butanol und Toluol, es ist erforderlich, dass der Methanolgehalt möglichst klein ist:
350 g (3,93 mol) 2-Amino-1-butanol werden mit 92 g (1,70 mol) festem Natrium-methanolat und 195 g Toluol versetzt. Bei einem Druck von 150 mbar werden an einer Kolonne mit 5 Trennstufen 171 g Destillat abdestilliert. Der Destillationssumpf fällt in Form einer homogenen Mischung an und weist einen Methanolgehalt von 2,22% auf.

## Patentansprüche

1. Verfahren zur Herstellung von Lösungen von Alkalimetallsalzen der allgemeinen Formel (I)
MOYXR¹ (I)
worin:
- M steht für Li, Na, K, Rb oder Cs,
- Y steht für (C₁-C₈)-Alkylen, wobei dieser unsubstituiert oder einfach oder mehrfach mit (C₁-C₈)-Alkoxy, (C₁-C₈)-Haloalkyl, Halogen, NH₂, NH(C₁-C₈)-Alkyl, N[(C₁₋C₈)-Alkyl]₂ oder S-(C₁-C₈)-Alkyl substituiert ist,
- X steht für O, S, NR¹,
- R¹ steht für
- H,
- (C₁-C₈)-Alkyl,
- (C₂-C₈)-Alkenyl,
- (C₂-C₈)-Alkinyl,
- (C₃-C₈)-Cycloalkyl, wobei dieser unsubstituiert oder mit einem oder mehreren Halogenen und/oder N-, O-, P-, S-atomhaltigen Resten substituiert ist und/oder N-, O-, P-, S-atomhaltige Reste im Ring aufweist, die unsubstituiert oder einfach oder mehrfach mit (C₁-C₈)-Alkoxy, (C₁-C₈)-Haloalkyl, Halogen, NH₂, S-(C₁-C₈)-Alkyl, (C₁-C₈)-Acyl, (C₁-C₈)-Alkyl substituiert sind,
- (C₆-C₁₈)-Aryl, wobei dieser unsubstituiert oder einfach oder mehrfach mit (C₁-C₈)-Alkoxy, (C₁-C₈)-Haloalkyl, Halogen, NH₂, S-(C₁-C₈)-Alkyl, (C₁-C₈)-Acyl, (C₁-C₈)-Alkyl substituiert ist,
- (C₇-C₁₉)-Aralkyl,
- (C₃-C₁₈)-Heteroaryl, wobei dieser unsubstituiert oder einfach oder mehrfach mit (C₁-C₈)-Alkoxy, (C₁-C₈)-Haloalkyl, Halogen, NH₂, S-(C₁-C₈)-Alkyl, (C₁-C₈)-Acyl oder (C₁-C₈)-Alkyl substituiert ist,
- (C₄-C₁₉)-Heteroaralkyl,
- ((C₁-C₈)-Alkyl)₁₋₃-(C₃-C₈)-Cycloalkyl,
- ((C₁-C₈)-Alkyl)₁₋₃-(C₆-C₁₈)-Aryl,
- ((C₁-C₈)-Alkyl)₁₋₃-(C₃-C₁₈)-Heteroaryl,
in einem Lösemittelgemisch aufweisend den korrespondierenden Alkohol der allgemeinen Formel (I) durch Umsetzung des korrespondieren Alkohols der allgemeinen Formel (I) mit dem entsprechenden Alkalimetallsalz eines Alkohols der allgemeinen Formel (II)
MOR² (II)
worin:
M die oben angegebene Bedeutung annimmt und
R² bedeutet Methyl, Ethyl, Propyl, Isopropyl, sec-, Iso- oder tert.-Butyl,
oder durch Umsetzung des korrespondierenden Alkohols der allgemeinen Formel (I) mit dem entsprechenden Alkalimetallhydroxid,
**dadurch gekennzeichnet,**
**dass** eine Teilmenge des freigesetzten Alkohols der allgemeinen Formel (II) oder des freigesetzten Wassers zunächst ohne Zusatz eines Schleppmittels und anschließend die Restmenge unter Zuhilfenahme mindestens eines weiteren organischen Lösemittels als Schleppmittel aus der Reaktionsmischung destilliert wird.

2. Verfahren nach Anspruch 1,
**dadurch gekennzeichnet,**
**dass** als weiteres organisches Lösemittel ein Schleppmittel ausgewählt wird aus der Gruppe bestehend aus, linearen oder cyclischen aliphatischen Kohlenwasserstoffen, aromatischen Kohlenwasserstoffen, Verbindungen des Typs der allgemeinen Formel (III)
R¹(XY)ₙXR¹ (III)
worin
n = 1 oder 2 ist,
R¹ jeweils unabhängig voneinander, X jeweils unabhängig voneinander und Y die in Anspruch 1 angegebene Bedeutung annehmen,
und Alkoholen der allgemeinen Formel (IV)
R³OH (IV)
worin:
R³ = (C₄-C₈)-Alkyl,
(C₂-C₈)-Alkenyl,
(C₂-C₈)-Alkinyl oder
(C₃-C₈)-Cycloalkyl wobei dieser unsubstituiert oder mit einem oder mehreren Halogenen und/oder N-, O-, P-, S-atomhaltigen Resten substituiert ist und/oder N-, O-, P-, S-atomhaltige Reste im Ring aufweist, die unsubstituiert oder einfach oder mehrfach mit (C₁-C₈)-Alkoxy, (C₁-C₈)-Haloalkyl, Halogen, NH₂, S-(C₁-C₈)-Alkyl, (C₁-C₈)-Acyl, (C₁-C₈)-Alkyl substituiert sind,
ist.

3. Verfahren nach Anspruch 1 und/oder 2,
**dadurch gekennzeichnet,**
**dass** die Destillationen bei Drücken von 10 mbar bis 15 bar durchgeführt werden.

4. Verfahren nach mindestens einem der vorhergehenden Ansprüche,
**dadurch gekennzeichnet,**
**dass** 10 - 90 Vol.-% des freigesetzten Alkohols (II) oder Wassers ohne Schleppmittel abdestilliert werden.

## Claims

1. Process for preparing solutions of alkali metal salts of the formula (I)
MOYXR¹ (I)
where
- M is Li, Na, K, Rb or Cs,
- Y is (C₁-C₈)-alkylene which is unsubstituted or monosubstituted or polysubstituted by (C₁₋C₈)-alkoxy, (C₁-C₈) -haloalkyl, halogen, NH₂, NH(C₁-C₈)-alkyl, N[(C₁-C₈)-alkyl]₂ or S-(C₁-C₈)-alkyl,
- X is O, S, NR¹,
- R¹ is
- H,
- (C₁-C₈)-alkyl,
- (C₂-C₈)-alkenyl,
- (C₂-C₈)-alkynyl,
- (C₃-C₈)-cycloalkyl which is unsubstituted or substituted by one or more halogens and/or N-, O-, P-, S-containing radicals and/or has N-, O-, P-, S-containing radicals in the ring, which are unsubstituted or monosubstituted or polysubstituted by (C₁-C₈) -alkoxy, (C₁-C₈)-haloalkyl, halogen, NH₂, S- (C₁-C₈) -alkyl, (C₁-C₈)-acyl, (C₁-C₈)-alkyl,
- (C₆-C₁₈)-aryl which is unsubstituted or monosubstituted or polysubstituted by (C₁-C₈)-alkoxy, (C₁-C₈)-haloalkyl, halogen, NH₂, S-(C₁-C₈)-alkyl, (C₁-C₈)-acyl, (C₁-C₈)-alkyl,
- (C₇-C₁₉)-aralkyl,
- (C₃-C₁₈)-heteroaryl which is unsubstituted or monosubstituted or polysubstituted by (C₁-C₈)-alkoxy, (C₁₋C₈)-haloalkyl, halogen, NH₂, S-(C₁-C₈)-alkyl, (C₁-C₈)-acyl or (C₁-C₈)-alkyl,
- (C₄-C₁₉)-heteroaralkyl,
- ((C₁-C₈)-alkyl)₁₋₃-(C₃-C₈) -cycloalkyl,
- ((C₁-C₈)-alkyl)₁₋₃-(C₆-C₁₈) -aryl,
- ((C₁-C₈)-alkyl)₁₋₃-(C₃-C₁₈) -heteroaryl,
in a solvent mixture comprising the corresponding alcohol of the formula (I) by reacting the corresponding alcohol of the formula (I) with the corresponding alkali metal salt of an alcohol of the formula (II)
MOR² (II)
where
M is as defined above and
R² is methyl, ethyl, propyl, isopropyl, sec-butyl, isobutyl or tert-butyl,
or by reacting the corresponding alcohol of the formula (I) with the corresponding alkali metal hydroxide,
**characterized in that**
part of the liberated alcohol of the formula (II) or the liberated water is distilled from the reaction mixture firstly without addition of an entrainer and the remainder is subsequently distilled from the reaction mixture with the aid of at least one further organic solvent as entrainer.

2. Process according to Claim 1,
**characterized in that**
the further organic solvent used is an entrainer selected from the group consisting of linear and cyclic aliphatic hydrocarbons, aromatic hydrocarbons, compounds of the formula (III)
R¹(XY)ₙXR¹ (III)
where
n = 1 or 2,
R¹, in each case independently of one another, X, in each case independently of one another, and Y are as defined in Claim 1,
and alcohols of the formula (IV)
R³OH (IV)
where
R³ is (C₄-C₈)-alkyl,
(C₂-C₈)-alkenyl,
(C₂-C₈)-alkynyl or
(C₃-C₈)-cycloalkyl which is unsubstituted or substituted by one or more halogens and/or N-, O-, P-, S-containing radicals and/or has N-, O-, P-, S-containing radicals in the ring, which are unsubstituted or monosubstituted or polysubstituted by (C₁-C₈)-alkoxy, (C₁-C₈)-haloalkyl, halogen, NH₂, S-(C₁-C₈)-alkyl, (C₁-C₈)-acyl, (C₁-C₈)-alkyl.

3. Process according to Claim 1 or 2,
**characterized in that**
the distillations are carried out at pressures of from 10 mbar to 15 bar.

4. Process according to at least one of the preceding claims,
**characterized in that**
10 - 90% by volume of the liberated alcohol (II) or water are distilled off without entrainer.

## Revendications

1. Procédé de préparation de solutions de sels de métaux alcalins de formule générale (I)
MOYXR¹ (I)
dans laquelle :
- M représente un Li, un Na, un K, un Rb ou un Cs,
- Y représente un groupe alkylène en (C₁-C₈), celui-ci étant non substitué ou mono- ou poly-substitué par un groupe alcoxy en (C₁-C₈), un groupe halogénoalkyle en (C₁-C₈), un atome d' halogène, un groupe NH₂, un groupe NH(C₁-C₈)-alkyle, un groupe N [(C₁-C₈)-alkyle]₂ ou un groupe S-(C₁-C₈)-alkyle,
- X représente un atome d'oxygène, un atome de soufre, un groupe NR¹,
- R¹ représente
- un atome d'hydrogène,
- un groupe alkyle en (C₁-C₈),
- un groupe alcényle en (C₂-C₈),
- un groupe alcynyle en (C₂-C₈),
- un groupe cycloalkyle en (C₃-C₈), celui-ci étant non substitué ou substitué par un ou plusieurs atomes d'halogène et/ou radicaux azotés, oxygénés, phosphorés ou soufrés, et/ou renfermant des radicaux azotés, oxygénés, phosphorés ou soufrés dans le noyau, qui sont non substitués ou mono- ou poly-substitués par un groupe alcoxy en (C₁₋C₈), un groupe halogénoalkyle en (C₁-C₈), un atome d'halogène, un groupe NH₂, un groupe S-(C₁-C₈)-alkyle, un groupe acyle en (C₁-C₈), un groupe alkyle en (C₁-C₈),
- un groupe aryle en (C₆-C₁₈), celui-ci étant non substitué ou mono- ou poly-substitué par un groupe alcoxy en (C₁-C₈), un groupe halogénoalkyle en (C₁-C₈), un atome d'halogène, un groupe NH₂, un groupe S-(C₁₋C₈) -alkyle, un groupe acyle en (C₁-C₈), un groupe alkyle en (C₁-C₈),
- un groupe aralkyle en (C₇-C₁₉),
- un groupe hétéroaryle en (C₃-C₁₈), celui-ci étant non substitué ou mono- ou poly-substitué par un groupe alcoxy en (C₁-C₈), un groupe halogénoalkyle en (C₁-C₈), un atome d'halogène, un groupe NH2, un groupe S-(C₁-C₈)-alkyle, un groupe acyle en (C₁-C₈)
ou un groupe alkyle en (C₁-C₈),
- un groupe hétéroaralkyle en (C₄-C₁₉),
- un groupe ((C₁-C₈)-alkyl)₁₋₃-(C₃-C₈)-cycloalkyle,
- un groupe ((C₁-C₈)-alkyl)₁₋₃-(C₆-C₁₈)-aryle,
- un groupe ((C₁-C₈)-alkyl)₁₋₃-(C₃-C₁₈)-hétéroaryle,
dans un mélange de solvants comprenant l'alcool correspondant de formule générale (I), par réaction de l'alcool correspondant de formule générale (I) avec le sel de métal alcalin correspondant d'un alcool de formule générale (II)
MOR² (II)
dans laquelle :
M adopte la signification indiquée ci-dessus, et
R² représente un groupe méthyle, un groupe éthyle, un groupe propyle, un groupe isopropyle, un groupe sec-butyle, un groupe isobutyle ou un groupe tert-butyle,
ou par réaction de l'alcool correspondant de formule générale (I) avec l'hydroxyde de métal alcalin correspondant,
**caractérisé en ce qu'**une quantité partielle de l'alcool libéré de formule générale (II) ou de l'eau libérée est d'abord distillée sans addition d'un agent d'entraînement, puis la quantité restante est distillée du mélange réactionnelle à l'aide d'au moins un solvant organique supplémentaire en tant qu'agent d'entraînement.

2. Procédé selon la revendication 1, **caractérisé en ce que** l'on choisit, en tant que solvant organique supplémentaire, un agent d'entraînement parmi le groupe constitué d'hydrocarbures aliphatiques linéaires ou cycliques, d'hydrocarbures aromatiques, de composés du type de formule générale (III)
R¹(XY)ₙXR¹ (III)
dans laquelle
n vaut 1 ou 2,
R¹, dans chaque cas indépendamment, X, dans chaque cas indépendamment, et Y adoptent la signification indiquée dans la revendication 1,
et d'alcools de formule générale (IV)
R³OH (IV)
dans laquelle :
R³ représente un groupe alkyle en (C₄-C₈),
un groupe alcényle en (C₂-C₈),
un groupe alcynyle en (C₂-C₈) ou
un groupe cycloalkyle en (C₃₋C₈), celui-ci étant non substitué ou substitué par un ou plusieurs atomes d'halogène et/ou radicaux azotés, oxygénés, phosphorés ou soufrés et/ou renfermant des radicaux azotés, oxygénés, phosphorés ou soufrés, qui sont non substitués ou mono- ou poly-substitués par un groupe alcoxy en (C₁-C₈), un
groupe halogénoalkyle en (C₁₋C₈), un atome d'halogène, un groupe NH₂, un groupe S-(C₁₋C₈)-alkyle, un groupe acyle en (C₁-C₈), un groupe alkyle en (C₁-C₈).

3. Procédé selon les revendications 1 et/ou 2, **caractérisé en ce que** la distillation est réalisée à des pressions de 10 mbars à 15 bars.

4. Procédé selon au moins l'une des revendications précédentes, **caractérisé en ce que** de 10 à 90 % en volume de l'alcool (II) ou de l'eau libéré(e) sont distillés sans agent d'entraînement.
